(19) Europäisches Patentamt
European Patent Office
Office européen des brevets

(11) EP 0 669 308 B1

(12) **EUROPÄISCHE PATENTSCHRIFT**

(45) Veröffentlichungstag und Bekanntmachung des Hinweises auf die Patenterteilung:
**22.04.1998 Patentblatt 1998/17**

(51) Int. Cl.$^6$: **C07C 43/303**, C11B 9/00

(21) Anmeldenummer: **95100518.0**

(22) Anmeldetag: **17.01.1995**

# (54) Isolongifolanol-Derivate, ihre Herstellung und ihre Verwendung

Isolongifolanol derivatives, their preparation and their use

Dérivés d'isolongifolanol, leur préparation et leur usage

(84) Benannte Vertragsstaaten:
**DE FR GB**

(30) Priorität: **23.02.1994 DE 4406467**

(43) Veröffentlichungstag der Anmeldung:
**30.08.1995 Patentblatt 1995/35**

(73) Patentinhaber:
**Dragoco Gerberding & Co Aktiengesellschaft**
**D-37603 Holzminden (DE)**

(72) Erfinder:
- **Brunke, Ernst Joachim, Dr.**
  **37603 Holzminden (DE)**
- **Schatkowski, Dietmar**
  **37627 Stadtoldendorf (DE)**

(74) Vertreter:
**Eikenberg, Kurt-Rudolf, Dr. Dipl.-Chem.**
**Eikenberg & Partner**
**Schackstrasse 1**
**30175 Hannover (DE)**

(56) Entgegenhaltungen:
**DE-A- 4 138 732** **GB-A- 1 505 821**

- **'The Chemistry of the Ether Linkage' 1967 , INTERSCIENCE PUBLISHERS , LONDON Dokument in bezug auf der Klarheit der Patentansprüche genannt. * Seite 310 - Seite 311 ***

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 669 308 B1

**Beschreibung**

Es besteht ein ständiger Bedarf an Riechstoffen mit verbesserten Eigenschaften wie Geruchsqualität, Stabilität in technischen Anwendungen, Hautverträglichkeit sowie Umweltverträglichkeit. Im Sinne einer Öko-Bilanz sind Produkte anzustreben, die auf nachwachsenden Rohstoffen basieren und die nach Gebrauch biologisch gut abbaubar sind. Ein in relativ großen Mengen verfügbarer Rohstoff natürlichen Ursprungs ist das Sesquiterpen Longifolen **(1)**, das als Hauptkomponente im indischen Terpentinöl und als Nebenkomponente in zahlreichen anderen Terpentinölsorten sowie weiteren etherischen Ölen vorkommt.

Bereits vor zwei Jahrzehnten wurden eine Reihe von Folgeprodukten des Longifolens **(1)** hergestellt und gewisse Riechstoffeigenschaften beschrieben. G. Ohloff berichtet in seinem Buch "Riechstoffe und Geruchssinn" (Springer-Verlag, Berlin 1990, ISBN-Nr. 3-540-52560-2, Seiten 87 - 88) zusammenfassend, daß von Longifolen **(1)** mindestens 4 kommerzielle Riechstoffe und aus Longifolen durch Isomerisierung zugänglichen Isolongifolen **(2)** 13 Riechstoffe kommerziell genutzt werden. Über Chemie und Geruchseigenschaften der kommerziell wichtigen Derivate des Isolongifolens **(2)** berichteten G. Färber und H. Tan [G. Färber, Parfümerie + Kosmetik, 68, 18 (1987), H. Tan, Parfümerie + Kosmetik, 67, 564 (1986)]. Grundsätzlich gelten die aus Isolongifolen **(2)** erhaltenen Verbindungen im Vergleich zu ähnlichen Longifolen-Derivaten als die sensorisch wertvolleren Riechstoffe warm-holzigen Geruchstyps.

In unserer eigenen EP-A-0 543 470 sind cyclische Isolongifolanon-Ketale der allgemeinen Formel **B**

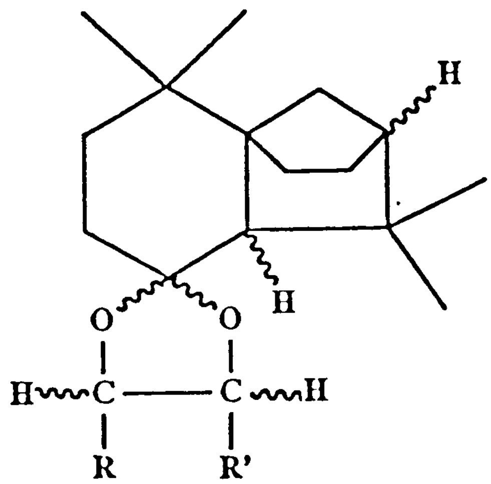

**B**

(in der die geschlängelten Linien α- und β-Konfigurationen sowie R und R' Wasserstoff- bzw Methyl- oder Ethylreste bedeuten) beschrieben. Diese Ketale sind wertvolle Riechstoffe, die stark holzige Geruchseigenschaften mit blumig-frischen Effekten und samtigem Moos-/Ambra-Charakter besitzen.

Des weiteren sind in der GB-A-1 505 821 Isolongifolanol und dessen niedere Ester (Acylgruppen mit bis zu 6 C-Atomen) als nützliche Riechstoffe beschrieben, wobei auch auf die α/β-Isomerie eingegangen wird. Diese Verbindungen besitzen stark und trocken holzige, an Cedrylacetat und Vetiverylacetat erinnernde Geruchseigenschaften und sind besonders gut für Parfum-Kompositionen mit blumigem oder citrus-holzigem Charakter geeignet.

Einige der vorgenannten Riechstoffe werden auf dem folgenden Reaktionsweg erhalten: Das durch Umsetzung von Isolongifolen **(2)** mit Persäuren zugängliche Epoxid **3** läßt sich in bekannter Weise in Gemische der epimeren Ketone **4a/4b** überführen, wobei diese Ketongemische in Abhängigkeit von den Produktionsbedingungen Unterschiedliche Isomerenverteilung und Geruchseffekte aufweisen können. Das Keton **4a** entsteht bevorzugt unter kinetischer Reaktionskontrolle, während das Keton **4b** das thermodynamisch stabilere Epimere ist. Die Verbindungen **3** sowie **4a/4b** sind kommerzielle Riechstoffe.

Entsprechend dem Stand der Technik gilt dieses Gebiet der Riechstoffchemie als besonders gut untersucht. Neben einigen Longifolen- und Isolongifolen-Derivaten mit Riechstoffeigenschaften sind eine größere Zahl von Derivaten der genannten Sesquiterpene bekannt, die keinen oder keinen wesentlichen olfaktorischen Wert besitzen. Es ist aus diesem Grund besonders überraschend, daß nunmehr auf dem Gebiet der Isolongifolen-Derivate die hier beschriebenen neuen Verbindungen der allgemeinen Formel **A**

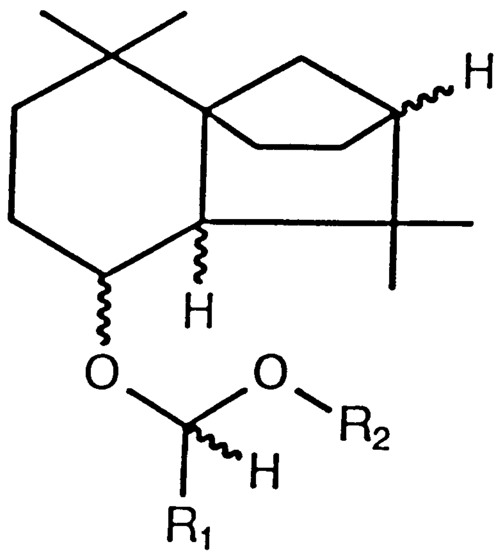

A

(in der die geschlängelten Linien α- und β-Konfiguration sowie $R_1$ Wasserstoff bzw. Methyl- oder Ethylreste und $R_2$ Methyl- oder Ethylreste bedeuten) aufgefunden werden konnten, die über ganz eigenständige geruchliche Eigenschaften verfügen und sich hierin deutlich von den bekannten Riechstoffen aus Isolongifolen **(2)** abheben und diese auch übertreffen. Die Isolongifolen-Derivate der allgemeinen Formel **A** besitzen Geruchseigenschaften vom Holz-Typ und vermitteln gleichzeitig einen strahlenden, starken Effekt, der ganz unterschiedliche Parfümnoten verstärkt und ihre Duftwirkung verlängert.

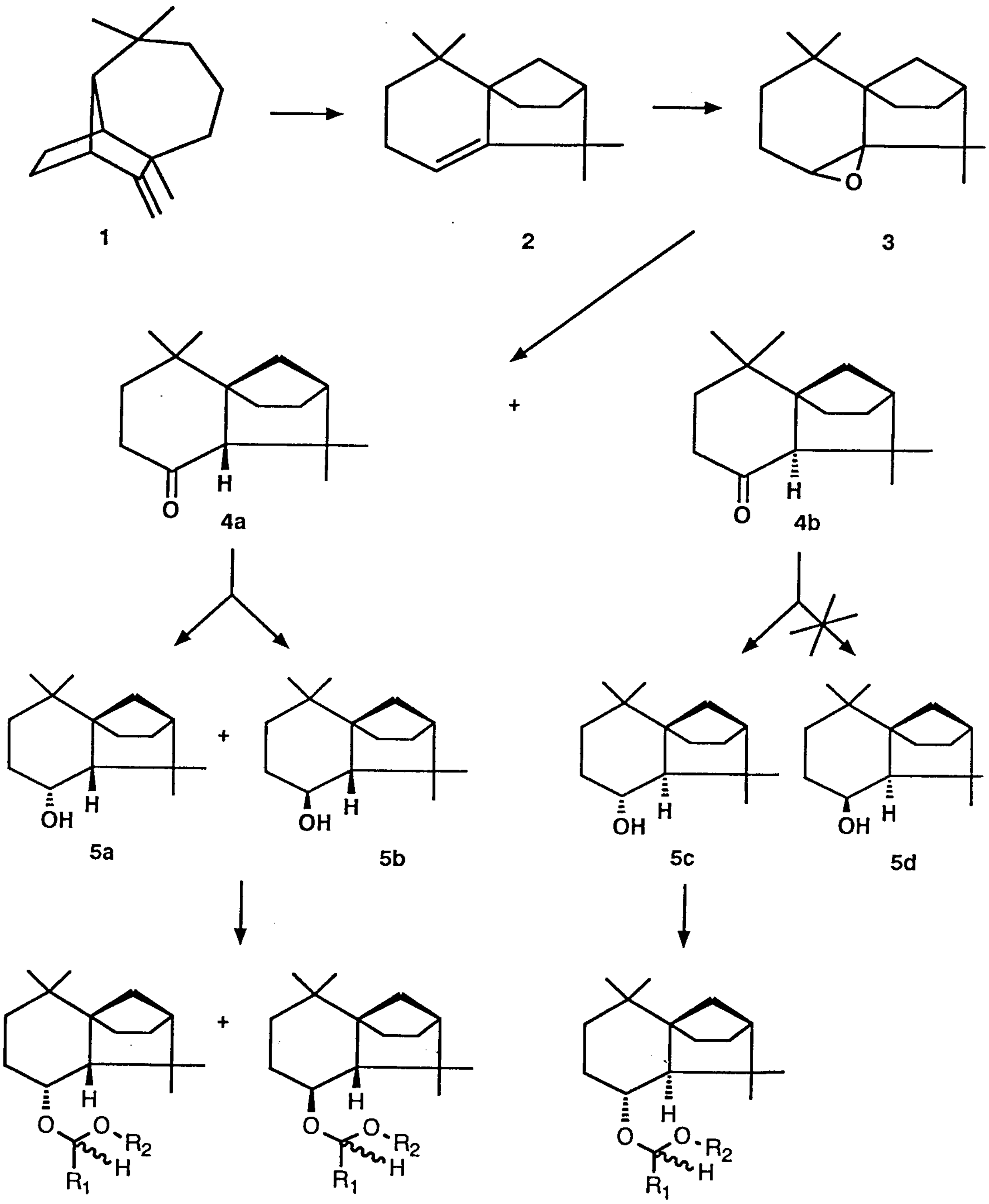

1 2 3

4a + 4b

5a + 5b 5c 5d

6a + 6b 6c

| | | |
|---|---|---|
| 6a $R_1$ = H, $R_2$ = Me | 6b $R_1$ = H, $R_2$ = Me | 6c $R_1$ = H, $R_2$ = Me |
| 7a $R_1$ = H, $R_2$ = Et | 7b $R_1$ = H, $R_2$ = Et | 7c $R_1$ = H, $R_2$ = Et |
| 8a $R_1$ = Me, $R_2$ = Et | 8b $R_1$ = Me, $R_2$ = Et | 8c $R_1$ = Me, $R_2$ = Et |

Zur Herstellung der Verbindungen der allgemeinen Formel **A** wurde Longifolen **(1)** in bekannter Weise durch Behandeln mit einem Gemisch aus Essigsäure und Schwefelsäure [U.R. Nayak, S. Dev, Tetrahedron 8, 42 - 48 (160)] oder mit Bortrifluorid-Etherat [R.E. Beyler, G. Okrisson, J. Org. Chem., 30, 2838 - 2839 (1965)] zu Isolongifolen **(2)** isomerisiert. Das aus Isolongifolen **(2)** erhaltene Epoxid **3** [L.K. Lala, J.B. Hall, J. Org. Chem., 35, 1172 (1970), I.R. Prahlad, R. Ranganathan, W.R. Nayak, T.S. Santhanakrishnan, S. Dev, Tetrahedron Lett., 8, 417 (1964)] wurde in bekannter Weise in das Gemisch der epimeren Ketone **4a/b** umgewandelt. [R. Ranganathan, W.R. Nayak, T.S. Santhanakrishnan, S. Dev, Tetrahedron, 26, 621 (1970)]. Die Strukturzuordnung erfolgte aufgrund der NMR-Meßergebnisse von J. Bombarda, J. Smadja, E.M., Gaydou, J.-Y. Conan und R. Fauré, J. Agric. Food Chem. 42, 138-142 (1994). Bei der Epoxid-Ringöffnung entstand unter kinetischer Kontrolle ein Ketongemisch, **4a/b**. Es ist bekannt, daß das Keton **4a** unter dem Einfluß basischer Katalysatoren oder durch thermische Belastung in das thermodynamisch stabilere Keton **4b** isomerisiert werden kann. In Abhängigkeit von den Reaktionsbedingungen werden Gleichgewichtsgemische aus den Ketonen **4a/4b** erhalten. Die Ketone **4a** und **4b** können in reiner Form oder als Gemisch in an sich bekannter Weise zu den epimeren Alkoholen **5a/b/c** umgesetzt werden [L.K. Lala, J. Org. Chem., 36, 2560 - 2561 (1971)]. Der Alkohol **5d** ist bisher nicht beschrieben worden und konnte auch im Verlauf unserer Arbeiten nicht nachgewiesen werden.

Die Alkohole **5a/b/c** wurden als Gemisch oder in reiner Form mit den symmetrisch substituierten Acetalen $R_2$-O-$CHR_1$-O-$R_2$ umgesetzt. Hierbei wurden die gemischten Acetale der Formeln **6a/b/c**, **7a/b/c** bzw. **8a/b/c** als Gemische bzw. in reiner Form erhalten. Für **5a** wurden die von J. Bombarda et. al. [J. Agric. Food Chem. 42, 136-142 (1994)] revidierte Strukturformel übernommen; die Strukturformeln **5b/c** wurden von uns den nach gegebener Vorschrift erhaltenen Alkoholen in Analogie zugeordnet.

Ausgehend von dem Keton **4a** wurde unter Baseneinfluß oder durch thermische Belastung wie erwartet das thermodynamisch stabilere Keton **4b** gewonnen. Dessen Reduktion führte wiederum nicht zu einem Gemisch der erwarteten epimeren Alkohole **5c/d**, sondern lediglich zum kristallinen Alkohol **5c**. Aus **5c** wurden durch Umsetzen mit den vorgenannten symmetrisch substituierten Acetalen die gemischten Acetale **6c** und **7c** jeweils in reiner Form erhalten.

Aus dem Isolongifolanon **4a** wurden durch Reduktion die epimeren Alkohole **5a** und **5b** erhalten und jeweils durch chromatographische Methoden isoliert. Umsetzen mit den vorgenannten symmetrischen Acetalen ergab jeweils die Acetale **6a**, **7a** sowie **6b**, **7b** in reiner Form. Die spektroskopische Charakterisierung und die Bestimmung der olfaktorischen Eigenschaften konnte somit anhand der reinen Substanzen vorgenommen werden.

Wahlweise kann ein aus dem Isolongifolen-Epoxid **3** gewonnenes Gemisch der Ketone **4a/b** zu einem Gemisch der diastereomeren Alkohole **5a/b/c** reduziert werden, die dann durch Umsetzung mit den symmetrischen Acetalen $R_2$-O-$CHR_1$-O-$R_2$ ein Gemisch der Acetale **6a/b/c** bzw. **7a/b/c** ergeben. In Abhängigkeit von den gewählten Reaktionsbedingungen können hierbei die epimeren Ketone **4a** und **4b** in unterschiedlichen Mengenverhältnissen anfallen, so daß nach Reduktion die diastereomeren Alkohole **5a/b/c** ebenfalls in unterschiedlichen Mengenverhältnissen vorliegen. Die stereochemischen Verhältnisse bleiben bei der Umsetzung mit den symmetrischen Acetalen $R_2$-O-$CHR_1$-O-$R_2$ im wesentlichen unverändert, so daß die neuen gemischten Acetale der Formel **A** in diesem Fall als Diastereomerengemische vorliegen.

Die neuen gemischten Acetale der Formel **A** besitzen jeweils in reiner Form oder als Steroisomeren-Gemische originelle Riechstoffeigenschaften und können in reiner Form oder als Diastereomerengemisch vorteilhaft als Riechstoffe bzw. Bestandteile von Parfümölen eingesetzt werden.

**Beispiel 1**

Herstellung von Isolongifolen **(2)**

Zu einer auf 60 °C erhitzten Lösung aus 360 g Toluol und 40 g (0.28 mol) $BF_3$-Etherat wurden innerhalb von 60 min 816 g (3.16 mol) Longifolen **(1)** (80 %, ex Terpentinöl indisch, $[\alpha]_D$ = + 39.4°) zugetropft, 3 h bei 100 °C nachgerührt, danach auf Raumtemperatur heruntergekühlt und mit Sodalösung und Wasser neutralgewaschen. Nach Trocknung über $Na_2SO_4$ wurde das Lösungsmittel unter vermindertem Druck abdestilliert. Es verblieben 800 g (74 %ig nach GC) Rohprodukt.

Gaschromatogramm (Shimadzu GC 14A, DBWAX-30 N, 30 m, 100° - 240 °C, 10 °C/min); $t_R$ = 5,16 min, 74 % **2**.

**Beispiel 2**

Herstellung von Isolongifolanon-Gemisch **4a/b** (96:4)

In einem Dreihalskolben mit Rückflußkühler und Tropftrichter wurden 800 g (2.90 mol) Isolongifolen **(2)** (74 %ig nach GC) aus Beispiel 1 und 276 g (6 mol) Ameisensäure vorgelegt und auf 60 - 70 °C erhitzt. Anschließend wurden über einen Zeitraum von 1 Std. 500 g (5.67 mol) $H_2O_2$ (35 %ig) zugetropft. Nach 3 Std. Rühren bei 80 - 85 °C wurde auf Raumtemperatur heruntergekühlt und aufgearbeitet. Die abgetrennte organische Phase wurde mit Sodalösung und Wasser neutralgewaschen, über $Na_2SO_4$ getrocknet und das Lösungsmittel unter reduziertem Druck abdestilliert. Es verblieben 804 g Rohprodukt.

GC: **4a** (71 %), **4b** (3,1 %) [96:4]

**Beispiel 3**

C-3-Epimerisierung von Isolongifolanon **(4a/4b)**

In einem 1 l-Dreihalskolben wurden 220 g (1 mol) Ketongemisch **4a/b** aus Beispiel 2, 200 g Methanol, 10 g NaOH (0.125 mol) vorgelegt und insgesamt 8 h unter Rückfluß gerührt. Anschließend wurde mit 7,5 g (0.125 mol) Eisessig versetzt und abgekühlt, das Lösungsmittel unter vermindertem Druck abdestilliert, der Rückstand in $H_2O$ aufgenommen, mit 100 g Ether versetzt und die org. Phase abgetrennt. Danach wurden die Wässer mit 100 g Ether extrahiert, die vereinigten org. Phasen mit Sodalösung und Wasser gewaschen, über $Na_2SO_4$ getrocknet und vom Lösungsmittel unter vermindertem Druck befreit. Es verblieben 212 g Rohprodukt als dunkelbraunes Öl.

GC (Bedingungen siehe Beispiel 1): **4a** (6,0 %), **4b** (69,1 %) [8:92]

**Beispiel 4**

Herstellung von Isolongifolanol-Gemisch **5a/b/c**

In einem Dreihalskolben mit Rückflußkühler und Tropftrichter wurden 800 g (2.90 mol) Isolongifolanon aus Beispiel 2 und 400 g Methanol vorgelegt. Anschließend wurden über einen Zeitraum von 1 h bis maximal 50 °C eine Lösung aus 250 g Wasser, 4 g NaOH und 37,8 g (1 mol) $NaBH_4$ zugetropft und 4 h bei einer Temperatur von 40 - 50 °C nachgerührt. Danach destillierte man das Lösungsmittel unter vermindertem Druck ab, nahm den Rückstand in 500 g Wasser auf und trennte die organische Phase ab.

Die abgetrennten Wässer wurden vereinigt und mit 200 g Hexan extrahiert. Die vereinigten organischen Phasen wurden mit Wasser neutralgewaschen, über $Na_2SO_4$ getrocknet und unter vermindertem Druck vom Lösungsmittel befreit. Es verblieben 768 g Rohprodukt.

GC (Bedingungen siehe Beispiel 1): **5a** (54,9 %), **5b** (15,3 %), **5c** (1 %)
GC/MS: HP 5985 A DBWAX-60 N, 60 m, 60 ° - 240 °C, 4 °C/min
**5a** Rt = 38,47 min.

MS (70 eV): m/e (%) = 222 (1, $M^+$), 189 (100), 161 (50), 133 (33), 125 (23), 119 (38), 105 (54), 91 (63), 55 (38), 41 (53).

**5b** Rt = 39,12 min.

MS (70 eV): m/e (%) = 222 (42, $M^+$), 207 (88), 166 (54), 151 (66), 123 (75), 109 (72), 95 (67), 81 (62), 55 (73), 41 (100)

**5c** Rt = 39,98 min.

MS (70 eV): m/e (%) = 222 (26, $M^+$), 207 (100), 189 (33), 166 (55), 137 (34), 123 (45), 109 (31), 69 (31), 41 (49)

**Beispiel 5**

Gewinnung von Isolongifolanol **5a**

1 g des aus Beispiel 4 gewonnenen rohen Isolongifolanol **5a/b/c** wurden durch präparative Chromatographie gereinigt.

Chromatographiebedingungen: 150 g Kieselgel 60, Korngröße 0.04 - 0.063 mm (Fa. Merck, Art.-Nr. 9385)

| | |
|---|---|
| Laufmittel: | Benzin/Essigester = 9/1 |
| Einwaage: | 1 g |
| Ausbeute: | 185 mg farblose Kristalle,<br>GC (Bedingungen siehe Beispiel 1): **5a** (97 %)<br>Smp.: 126 - 128 °C |

GC/MS: Bedingungen siehe Beispiel 4
**5a** Rt = 38,47 min.

| | |
|---|---|
| MS (70 eV): m/e (%) = | 222 (1, $M^+$), 189 (100), 161 (50), 133 (33), 125 (23), 119 (38), 105 (54), 91 (63), 55 (38), 41 (53). |
| $^{13}$C-NMR ($CDCl_3$), Varian VXR-300): $\delta$ [ppm]: | 22.07, 23.43, 27.57, 32.98 ($\underline{C}H_3$), 22.67, 25.58, 32.10, 37.91 ($\underline{C}H_2$), 48.20, 52.17, 68.12 ($\underline{C}H$), 33.66, 37.65, 54.60 ($\underline{C}$). |

**Beispiel 6**

Herstellung von Isolongifolanol **5c**

In einem Dreihalskolben mit Rückflußkühler und Tropftrichter wurden 212 g (0.72 mol) Isolongifolanon aus Beispiel 3 und 10 g Methanol vorgelegt. Anschließend wurde über einen Zeitraum von 0,5 h bis max. 50 °C eine Lösung aus 70 g Wasser 1 g NaOH und 9,5 g (0.25 mol) $NaBH_4$ zugetropft und 3 h bei 40-50 °C nachgerührt. Danach wurde das Lösungsmittel unter vermindertem Druck abdestilliert, der Rückstand in 100 g Wasser und 100 g Ether aufgenommen, die org. Phase abgetrennt, die Wässer nochmals mit 100 g Ether extrahiert und die vereinigten org. Phasen mit Wasser neutral gewaschen, über $Na_2SO_4$ getrocknet und das Lösungsmittel unter vermindertem Druck abdestilliert. Es verblieben 198 g öliges, erstarrendes Produkt.

GC (Bedingungen siehe Beispiel 1): **5a** (2,1 %); **5b** (3,1 %), **5c** (65,1 %)

Durch Umkristallisieren von

| | |
|---|---|
| 100 g | des so erhaltenen Rohproduktes aus |
| 200 g | Hexan Fraktion 63/80 wurden |
| 53 g | **5c** als farblose Kristalle erhalten, Smp. 126 - 128 °C. |

GC (Bedingungen siehe Beispiel 1): **5c** (95,1 %).

**Beispiel 7**

Herstellung eines Gemisches von Formaldehyd-Methyl-Isolongifolanylacetalen **(6a/b/c)**

In einem Dreihalskolben mit Tropftrichter, 20 cm Glasfüllkörperkolonne und Kolonnenkopf wurden 111 g (0,36 mol) Isolongifolanol aus Beispiel 3 [GC: **5a** (54,9 %), **5b** (15,3 %), **5c** (1 %)], 2 g para-Toluolsulfonsäure und 228 g (3 mol) Formaldehyddimethylacetal vorgelegt und zum Sieden erhitzt. Anschließend wurden über einen Zeitraum von 8 h insgesamt 150 g eines azeotropen Gemisches aus Methanol/Formaldehyddimethylacetal abdestilliert, wobei gleichzeitig 152 g (2 mol) Formaldehyddimethylacetal in 8 h zugetropft wurden. Anschließend wurden weitere 16 h unter Rückfluß nachgerührt, auf Raumtemperatur heruntergekühlt und mit Sodalösung und Wasser neutralgewaschen. Es verblieben 117 g Rohprodukt.

GC (Bedingungen siehe Beispiel 1): **6a** (49,1 %), **6b** (13,3 %), **6c** (0,9 %)
Destillation über eine 15 cm Vigreux-Kolonne ergab 107 g **6a**/**b**/**c**,
$Kp_2$ mm 120 - 150 °C
GC (Bedingungen siehe Beispiel 1): **6a** (51,4 %), **6b** (15,6 %), **6c** (1,1 %).

Anschließende Feindestillation über eine 50 cm Metallfüllkörperkolonne ergab 67 g $Kp_{2mm}$ 135 - 139 °C

GC (Bedingungen siehe Beispiel 1): **6a** (75,3 %), **6b** (20,8 %), **6c** (2,3 %).

| | |
|---|---|
| D 20/4 | = 0.9854 |
| n 20/D | = 1.4753 |
| [$\alpha$] 20/D | = + 2.1 ° |

**Beispiel 8**

Herstellung eines Gemisches von Formaldehyd-Ethyl-Isolongifolanylacetalen **(7a/b/c)**

In einem Dreihalskolben mit Tropftrichter, 20 cm Glasfüllkörperkolonne mit Kolonnenkopf wurden 500 g (1,60 mol) Isolongifolanol aus Beispiel 3 [GC: **5a** (54,9 %), **5b** (15,3 %), **5c** (1 %)], 5 g para-Toluolsulfonsäure und 342 g (3 mol) Formaldehyddiethylacetal vorgelegt und zum Sieden erhitzt. Anschließend wurden über einen Zeitraum von 8 h insgesamt 212 g eines azeotropen Gemisches aus Ethanol/Formaldehyddiethylacetal abdestilliert, wobei gleichzeitig 208 g (2 mol) Formaldehyddiethylacetal in 8 h zugetropft wurden. Anschließend wurde auf Raumtemperatur heruntergekühlt und mit Sodalösung und Wasser neutralgewaschen. Es verblieben 513 g Rohprodukt.

GC (Bedingungen siehe Beispiel 1): **7a** (52,3 %) **7b** (14,9 %), **7c** (0,8 %)

Eine Destillation über eine 15 cm Vigreux-Kolonne ergab 493 g rohes **7a/b/c**, $Kp_{2mm}$ 115 - 158 °C

GC (Bedingungen siehe Beispiel 1): **7a** (53,1 %), **7b** (15,3 %), **7c** (0,7 %)

Anschließende Feindestillation über eine 50 cm Metallfüllkörperkolonne ergab 311 g $Kp_{2mm}$ 141 - 144 °C

GC (Bedingungen siehe Beispiel 1)**:** **7a** (58,1 %), **7b** (22,0 %), **7c** (15,4 %)

| | |
|---|---|
| D 20/4 | = 0,9899 |
| n 20/D | = 1,4867 |
| [α] 20/D | = + 3,3° |

**Beispiel 9**

Herstellung von Formaldehyd-Methyl-Isolongifolanylacetal **6c**

5 g des aus Beispiel 5 erhaltenen Isolongifolanol **5c** wurden nach der in Beispiel 6 beschriebenen Weise zu 4,95 g rohem Formaldehyd-Methyl-Isolongifolanylacetal **6c** umgesetzt.

GC (Bedingungen siehe Beispiel 1): **5c**: 21,5 %; **6c**: 72,8 %

**Beispiel 10**

Isolierung von Formaldehyd-Methyl-Isolongifolanylacetal **6c**

1 g rohes Acetal aus Beispiel 8 [GC: **6c** (72,8 %)] wurden durch mehrfache Flash-Chromatographie (2 x) gereinigt. Chromatographiebedingungen: 200 g Kieselgel 60, Korngröße 0.04 - 0.063 mm (Fa. Merck, Art.-Nr. 9385)

| | |
|---|---|
| Laufmittel: | Benzin/Essigester = 98/2 |
| Einwaage: | 1 g |
| Ausbeute: | 195 mg, |
| | GC (Bedingungen siehe Beispiel 1): **6c** (89,2 %) |

GC/MS-Bedingungen siehe Beispiel 4
**6c** Rt = 37,79 min.

| | |
|---|---|
| MS (70 ev): m/e (%) = | 266 (20, $M^+$), 189 (31), 165 (33), 109 (56), 107 (25), 102 (26), 95 (43), 81 (34), 69 (25), 45 (100), |
| $^{13}$C-NMR ($CDCl_3$), Varian VXR-300): | δ [ppm]:24.85, 25.35, 26.70, 29.14, 55.66 ($\underline{C}H_3$), 25.35, 28.05, 31.16, 36.99, 37.20, 95.81 ($\underline{C}H_2$), 49.27, 57.00, 79.11 ($\underline{C}H$), 32.25, 40.64 (56.29 ($\underline{C}$). |

**Beispiel 11**

Herstellung von Formaldehyd-Ethyl-Isolongifolanylacetal **7c**

10 g des aus Beispiel 5 erhaltenen Isolongifolanol **5c** wurden nach der in Beispiel 7 beschriebenen Weise zu 9,8 g rohem Formaldehyd-Ethyl-Isolongifolanylacetal **7c** umgesetzt.

GC (Bedingungen siehe Beispiel 1): **5c**: 16,6 %; **7c**: 79,1 %

**Beispiel 12**

Isolierung von Formaldehyd-Ethyl-Isolongifolanylacetal **7c**

1 g rohes Acetal aus Beispiel 8 [GC: **7c** (79,1 %)] wurden durch Flash-Chromatographie gereinigt.
Chromatographiebedingungen: 150 g Kieselgel 60, Korngröße 0,04 - 0,063 mm (Fa. Merck, Art.-Nr. 9385)

| | |
|---|---|
| Laufmittel: | Benzin/Essigester = 95/5 |
| Einwaage: | 1 g |
| Ausbeute: | 495 mg; GC: **7c** (95,1 %) |

GC/MS: Bedingungen siehe Beispiel 4
**7c** Rt = 38,39 min.

| | |
|---|---|
| MS (70 eV): m/e (%) = | 280 (9, $M^+$), 204 (26), 165 (37), 116 (30), 109 (55), 95 (37), 81 (26), 69 (26), 59 (100), 31 (35). |
| $^{13}$C-NMR ($CDCl_3$), Varian VXR-300): | δ [ppm] = 15.07, 24.87, 25.34, 26.71, 29.15 ($\underline{C}H_3$), 26.34, 27.92, 31.17, 36.99, 37.20, 63.56, 94.13 ($\underline{C}H_2$), 49.28, 57.04, 78.80 ($\underline{C}H$), 32.28, 40.64, 56.71 ($\underline{C}$). |

**Beispiel 13**

Isolierung von Formaldehyd-Ethyl-Isolongifolanylacetal-Gemisch **7a/7b**

1 g rohes Acetal aus Beispiel 7 wurden durch präparative Gaschromatographie gereinigt.
Chromatographiebedingungen: GC: G/RA CAP 12, Aluminiumsäule-Carbowax 10 % Belegung, 3/9 Zoll, 200 °C isotherm; Gasfluß: 120 ml/min;

| | |
|---|---|
| Detektor: | W OL; Injektionen: 20 x 50 mg |
| Ausbeute: | 135 mg; GC (Bedingungen siehe Beispiel 1): **7a** (96,1%) 53 mg; GC (Bedingungen siehe Beispiel 1); **7b** (92,0%) |

GC/MS: Bedingungen siehe Beispiel 4
**7a** Rt = 35,15 min.

| | |
|---|---|
| MS (70 eV): m/e (%) = | 280 (7, $M^+$), 204 (56), 161 (40), 109 (35), 105 (40), 91 (43), 59 (100), 41 (40), 31 (46). |
| $^{13}$C-NMR ($CDCl_3$), Varian VXR-300): | δ [ppm]: 15.12, 22.35, 26.31, 27.54, 33.08 ($\underline{C}H_3$), 22.51, 25.50, 28.77, 32.80, 37.90, 63.52, 95.44 ($\underline{C}H_2$), 48.35, 52.12, 75.90 ($\underline{C}H$), 33.52, 37.55, 54.88 ($\underline{C}$). |

**7b** Rt = 37.22 min.

| | |
|---|---|
| MS (70 eV): m/e (%) = | 280 (7, $M^+$), 204 (47), 189 (19), 161 (22), 109 (35), 95 (31), 81 (24), 69 (23), 59 (100), 31 (38). |
| $^{13}$C-NMR ($CDCl_3$), Varian VXR-300): δ [ppm]: | 15.10, 21.16, 23.29, 26.46, 33.27 ($\underline{C}H_3$ ), 21.53, 26.46, 30.43, 36.48, 27.68, 63.55, 94.70 ($\underline{C}H_2$), 48.36, 53.79, 76.56 ($\underline{C}H$), 37.47, 56.41 ($\underline{C}$). |

**Beispiel 14**

Isolierung von Formaldehyd-Methyl-Isolongifolanylacetal **6a**

1 g des aus Beispiel 6 gewonnenen Gemisches von Formaldehyd-Methyl-Isolongifolanylacetalen **6a/b/c** wurden durch mehrfache Flash-Chromatograhie (3 x) gereinigt.

Chromatographiebedingungen: 150 g Kieselgel 60, Korngröße 0.04 - 0.063 mm (Fa. Merck, Art.-Nr. 9385)

| | |
|---|---|
| Laufmittel: | Benzin/Essigester = 95/5 |
| Einwaage: | 1 g |
| Ausbeute: | 85 mg |
| | GC (Bedingungen siehe Beispiel 1): **6a** (98 %) |

GC/MS-Bedingungen siehe Beispiel 4
**6a** Rt = 34.25 min.

| | |
|---|---|
| MS (70 eV): m/e (%) = | 266 (6, $M^+$), 204 (47), 189 (59), 161 (32), 109 (26), 105 (32), 91 (34), 81 (24), 45 (100), 41 (24). |
| $^{13}C$-NMR ($CDCl_3$), Varian VXR-300): | δ [ppm]: 22.40, 23.60, 27.55, 33.07, 55.70 (<u>C</u>$H_3$), 22.05, 25.50, 28.86, 32.82, 37.89, 97.08 (<u>C</u>$H_2$), 48.34, 52.13, 76.11 (<u>C</u>H), 33.50, 37.54, 54.87 (<u>C</u>). |

**Beispiel 15**

Isolierung von Formaldehyd-Methyl-Isolongifolanylacetal **6b**

3 g des aus Beispiel 6 gewonnenen Gemisches von Formaldehyd-Methyl-Isolongifolanylacetalen **6a/b/c** wurden durch mehrfache Flash-Chromatograhie (3 x) gereinigt.

Chromatographiebedingungen: 300 g Kieselgel 60, Korngröße 0.04 - 0.063 mm (Fa. Merck, Art.-Nr. 9385)

| | |
|---|---|
| Laufmittel: | Benzin/Essigester = 95/5 |
| Einwaage: | 3 g |
| Ausbeute: | 146 mg |
| | GC (Bedingungen siehe Beispiel 1): **6b** (92.3 %) |

GC/MS-Bedingungen siehe Beispiel 4
**6b** Rt = 36.56 min.

| | |
|---|---|
| MS (70 eV): m/e (%) = | 266 ($M^+$, 81, 234 (9), 204 (67), 189 (312), 161 (30), 109 (37), 95 (32), 81 (28), 69 (20), 45 (100). |
| $^{13}C$-NMR ($CDCl_3$), Varian VXR-300): | δ [ppm]: 21.17, 23.27, 26.46, 32.20, 55.70 (<u>C</u>$H_3$), 21.52, 26.46, 30.48, 36.48, 37.67, 96.29 (<u>C</u>$H_2$), 48.61, 53.75, 76.74 (<u>C</u>H), 37.44, 56.36 (<u>C</u>). |

**Beispiel 16**

Herstellung eines Gemisches von Acetaldehyd-Ethyl-Isolongifolanylacetalen **(8a/b/c)**

In einem Dreihalskolben mit Rückflußkühler und Tropftrichter wurden 70.2 g (0.25 mol) Isolongifolanol aus Beispiel 2 und 1 g HCl conz. vorgelegt. Anschließend tropft man über einen Zeitraum von 1 h bei 10 °C eine Lösung aus 253 g (1.14 mol) Isolongifolanol aus Beispiel 2 und 358 g (5.73 mol) Vinyl-Ethyl-Ether zu und rührt 48 h bei Raumtemperatur nach. Nach dieser Zeit wurde das Reaktionsgemisch einmal mit 100 g gesättigter Natriumhydrogencarbonat-Lösung und danach mit Wasser neutralgewaschen, über $Na_2SO_4$ getrocknet und unter vermindertem Druck vom überschüssigen Vinyl-Ethyl-Ether befreit. Es verblieben 318 g Rohprodukt. Destillation über eine 15 cm Vigreux-Kolonne ergab 297 g **8a/b/c**, $Kp_{2mm}$ 92 - 140 °C. Anschließende Feindestillation über eine 50 cm Metallfüllkörperkolonne ergab 147 g $Kp_{2mm}$ 135 - 139 °C.

GC (Bedingungen siehe Beispiel 1): Σ aus **8a/b/c** = ca. 92,5 %

D 20/4 = 0,9769
n 20/D = 1,4841
[α] 20/D = - 5,0°

GC/MS-Bedingungen siehe Beispiel 4
Rt 33.46 min.

MS (70 eV): m/e (%) = 276 (2), 266 (3), 221 (18), 203 (7), 177 (7), 149 (5), 73 (100), 45 (31), 31 (5).

Rt 34.42 min.

MS (70 eV): m/e (%) = 265 (1), 221 (20), 203 (7), 177 (7), 149 (5), 104 (3), 91 (4), 73 (100), 45 (30).

Rt 34.76 min.

MS (70 eV): m/e (%) = 279 (1), 265 (1), 121 (2), 205 (10), 149 (3), 109 (3), 95 (4), 73 (100), 45 (24), 41 (4).

Rt 36.00 min.

MS (70 eV: m/e (%) = 265 (1), 205 (4), 149 (3), 109 (2), 91 (2), 81 (3), 73 (100), 45 (22).

Rt 36.44 min.

MS (70 eV): m/e (%) = 279 (1), 205 (10), 149 (3), 109 (3), 95 (4), 81 (3), 73 (100), 45 (23).

Rt 37.42 min.

MS (70 eV): m/e (%) = 265 (1), 205 (5), 149 (3), 109 (2), 91 (2), 73 (100), 45 (23).

**Beispiel 17**

<u>Geruchsbeschreibungen der Acetale **6a/b/c bis 8a/b/c**</u>

Die Geruchsbewertung wurde von einem Expertengremium anhand 10 %iger ethanolischer Lösung unter Verwendung von Riechstreifen vorgenommen.

**6a/b/c** aus Beispiel 7:
holzig, würzig, krautig, leicht grün-metallisch, exaltierend.
**6a** aus Beispiel 14:
holzig, pudrig, exaltierend, warm-weich, schwächer als **6b**, stärker als **6c**.
**6b** aus Beispiel 15:
stark, würzig-pfeffrig, stark holzig-frisch, schwach krautigmetallisch.
**6c** aus Beispiel 10:
holzig, Fenchel-Note.
**7a/b/c** aus Beispiel 8:
<u>stark</u> holzig, exaltierend, pudrig, warm-fruchtig.
**7a** aus Beispiel 13:
säuerlich-holzig, exaltierend, schwach grün-staubig schwächer als **7b**, stärker als **7c**.
**7b** aus Beispiel 13:
<u>stark</u>, trocken-pudrig-würzig, grün, krautig, exaltierend.
**7c** aus Beispiel 12:
holzig, krautig, süßlich.
**8a/b/c** aus Beispiel 16:
holzig, trocken

**Beispiel 18**

Anwendungsbeispiel 1

Parfümöl für ein Damen-Parfum orientalischen Typs.

Durch Zugabe von 20 Teilen der Verbindung **7a/7b/7c** wird der orientalisch-blumige Duftcharakter deutlich verstärkt. Die Komposition erfährt einen "Lift"-Effekt und zeigt verbesserte Fixierung.

| | | |
|---|---|---|
| Orangenöl Guinea | 20 | - |
| Aldehyd C14 | 5 | - |
| Jasmin Base | 20 | - |
| Rosen Base | 10 | - |
| Amylzimtaldehyd | 20 | - |
| Hedione(R) (a) | 16 | - |
| Cyclopentadecanolide | 15 | - |
| Galaxolide(R) (b) | 10 | - |
| Lyral(R) (b) | 4 | - |
| Helional | 5 | - |
| Huminol 1 %(R) (c) | 2 | - |
| Tabanon 10 %(R) (c) | 2 | - |
| Parmanyl(R) (c) | 2 | - |
| **Verbindung 7a/b/c** | **200** | **-** |
| DPG | - | 200 |
| | 700 | 700 |

(a) Firmenich
(b) IFF
(c) DRAGOCO

**Beispiel 19**

Anwendungsbeispiel 2

Parfümöl für ein maskulines Fine Fragrance. Das Parfümöl der angegebenen Formel besitzt einen ausgeprägten frisch-krautigen Charakter mit citrisch-grünen Aspekten. Die Zugabe von 10 Teilen **7a/b/c** ergibt eine sehr gewünschte Harmonisierung unter gleichzeitiger Betonung von exaltierenden holzigen Aspekten. Alternative Zugabe von **6a/b/c** ergibt ebenfalls eine gewünschte Abrundung, jedoch mit Betonung trocken-holziger Aspekte.

| | | |
|---|---|---|
| Bergamottöl italienisch | 80 | - |
| Dihydromyrcenol | 20 | - |
| Geraniumöl BB | 10 | - |
| Linalool | 30 | - |
| Linalylacetat | 60 | - |
| Lavendelöl französisch | 30 | - |
| Isogalbanat | 10 | - |
| Isodamascon 10 % | 3 | - |
| Cyclogalbanat | 20 | - |
| Brahmanol F | 5 | - |
| Precyclemone B | 5 | - |
| Ambroxan | 20 | - |
| Hedione | 100 | - |
| Anisaldehyd 10 % | 7 | - |
| Coumarin | 20 | - |
| Limettenöl Tahiti | 40 | - |
| **Verbindung 7a/7b/7c** | **100** | **-** |
| DPG | - | 100 |
| | 740 | 740 |

(a) Firmenich
(b) IFF
(c) DRAGOCO

In beiden Fällen bewirkt die Zugabe von Isolongifolanylacetalen eine verbesserte Haftung der Hauptnoten.

## Patentansprüche

1. Isolongifolanol-Derivate, der allgemeinen Formel **A**, wobei geschlängelte Linien α- und β-Konfiguration sowie $R_1$ Wasserstoff bzw. Methyl- oder Ethylreste und $R_2$ Methyl- oder Ethylreste bedeuten.

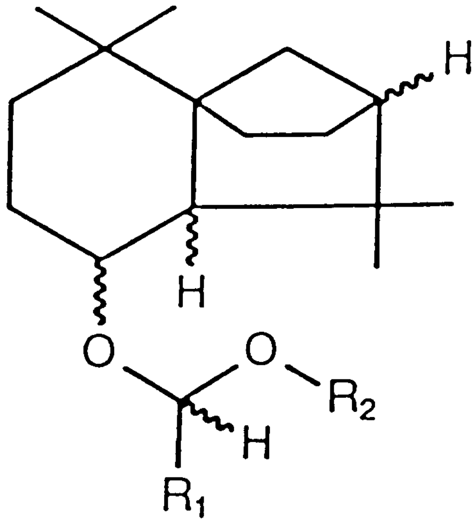

A

2. Verfahren zur Herstellung der Verbindungen der allgemeinen Formel **A**, dadurch gekennzeichnet, daß man in an sich bekannter Weise aus Longifolen erhaltenes Isolongifolen zu Isolongifolan-3-on oxidiert, dieses wahlweise unter Baseneinwirkung equilibriert und/oder zu Isolongifolan-3-ol reduziert und dieses dann mit einem symmetrischen Acetal der Formel $R_2$-O-$CHR_1$-O-$R_2$ umsetzt, worin $R_1$ Wasserstoff bzw. Methyl- oder Ethylreste sowie $R_2$ Methyl- oder Ethylreste sind.

3. Verwendung der Verbindungen der allgemeinen Formel **A** als Riechstoffe oder Bestandteile von Riechstoff-Mischungen bzw. Parfümölen zur Parfümierung von kosmetischen oder technischen Gebrauchsgütern.

**Claims**

1. Isolongifolanol derivatives of the general formula A, in which the wavy lines mean α and β configuration, $R_1$ means hydrogen, methyl or ethyl groups and $R_2$ means methyl or ethyl groups

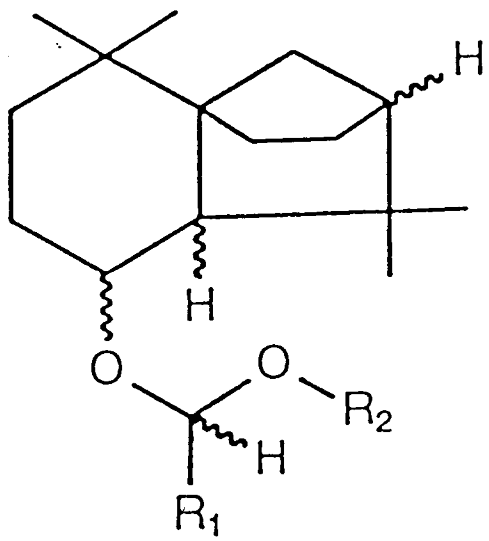

A

2. Process for production of compounds of the general formula A, characterised in that in a method known per se isolongifolene obtained from longifolene is oxidised to isolongifolane, which is then equilibrated with base extraction and/or reduced to isolongifolan-3-ol and this is then reacted with a symmetric acetal of the formula $R_2$-OCH$R_1$-O-$R_2$, in which $R_1$ is hydrogen, or methyl or ethyl group and $R_2$ is methyl or ethyl group.

3. Use of compounds of the general formula A as odorants or components or mixtures of odorants or perfume oils for the perfuming of cosmetic or technical consumer goods.

**Revendications**

1. Dérivés d'isolongifolanole de la formule générale A, les lignes sinueuses signifiant des configurations $\alpha$ et $\beta$ ainsi que $R_1$ des restes d'hydrogène ou de méthyle ou d'éthyle et $R_2$ des restes de méthyle ou d'éthyle.

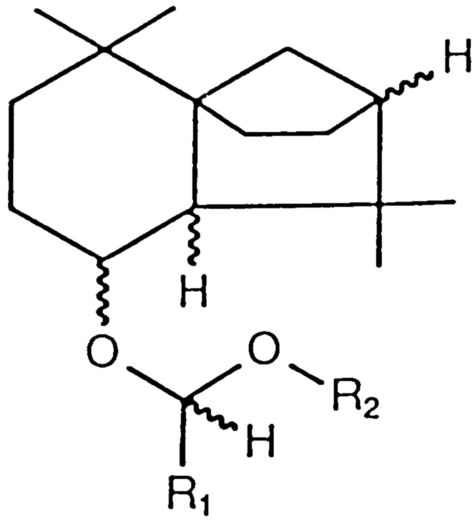

A

2. Procédé de fabrication des composés de la formule générale A, caractérisé en ce que l'on oxyde de manière connue en soi de l'isolongifolène obtenu à partir de longifolène en isolongifolan-3-one, on équilibre celui-ci au choix avec influence de la base et/ou on le réduit en isolongifolan-3-ol et on le transforme ensuite avec un acétal symétrique de la formule $R_2$-O-$CHR_1$-O-$R_2$, $R_1$ étant des restes d'hydrogène ou de méthyle ou d'éthyle et $R_2$ des restes de méthyle ou d'éthyle.

3. Utilisation des composés de la formule générale A comme matières odorantes ou parties composantes de mélanges de matières odorantes ou d'huiles pour parfum pour parfumer des articles de consommation cosmétiques ou techniques.